(19) Europäisches Patentamt
European Patent Office
Office européen des brevets

(11) Veröffentlichungsnummer: **0 122 388**
**B1**

(12) # EUROPÄISCHE PATENTSCHRIFT

(45) Veröffentlichungstag der Patentschrift: **06.11.85**

(51) Int. Cl.⁴: **C 07 C 69/716,** C 07 C 69/72, C 07 C 49/76, C 07 C 49/782, C 07 C 125/00, C 07 D 307/54 // C07D409/06, C07D241/44, C07D241/42, C07D253/06, C07C109/16, C07C149/46

(21) Anmeldenummer: **84101465.7**

(22) Anmeldetag: **13.02.84**

(54) **Verfahren zur Herstellung von vicinalen Polycarbonylverbindungen.**

(30) Priorität: **19.02.83 DE 3305804**

(43) Veröffentlichungstag der Anmeldung: **24.10.84 Patentblatt 84/43**

(45) Bekanntmachung des Hinweises auf die Patenterteilung: **06.11.85 Patentblatt 85/45**

(84) Benannte Vertragsstaaten: **BE CH DE FR GB IT LI**

(56) Entgegenhaltungen:
**DE - B - 1 207 379**

**CHEMISCHE BERICHTE, Band 115, Nr. 9, 9. September 1982, Weinheim, K. SCHANK et al. "Ozonspaltung von Sulfonium-Yliden"**
**Chemical Abstracts Band 92, Nr. 19, 12. Mai 1980, Columbus, Ohio, USA, W. ANDO et al. "Phtosensitized oxygenation of carbonyl stabilized sulfur and pyridinium ylides and related diazo compounds. Carbon-sulfur and nitrogen bond cleavages", Seite 546, Spalte 2, Abstract"**
**HOUBEN WEYL "Methoden der organischen Chemie", 4. Auflage, Band VII/2a, Teil 1, 1973, GEORG THIEME VERLAG, Stuttgart, Seiten 677-687**

(73) Patentinhaber: **BASF Aktiengesellschaft, Carl-Bosch-Strasse 38, D-6700 Ludwigshafen (DE)**

(72) Erfinder: **Koenig, Horst, Prof. Dr., Pariser Strasse 4, D-6700 Ludwigshafen (DE)**

**EP 0 122 388 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Diese Erfindung betrifft ein Verfahren zur Herstellung von vicinalen Polycarbonylverbindungen durch Oxidation von Schwefelyliden.

Vicinale Polycarbonylverbindungen, wie 1,2-Di- und 1,2,3-Tricarbonylverbindungen, sind wichtige Ausgangsstoffe für die Herstellung heterocyclischer Verbindungen, die z. B. als Wirkstoffe für Arzneimittel oder Pflanzenschutzmittel oder als Zwischenprodukte für die Herstellung von Farbstoffen verwendet werden (s. u. a. Chemical Reviews, 1975, Vol. 75, Nr. 2, S. 177 bis 202 und DE-OS Nr. 2710902, S. 25 bis 27).

Nach den bisher bekannten Methoden lassen sich vicinale Polycarbonylverbindungen durch eine Oxidation der Methylengruppen der entsprechenden β-Dicarbonylverbindungen, z. B. durch Behandlung mit Selendioxid (Helv. Chimica Acta *57*, Fasc. 7 (1974), S. 2201 bis 2208) mit Nitrosoverbindungen oder mit Diazoniumsalzen (Chemical Reviews, 1975, Vol. 75, Nr. 2, S. 178 und 184) herstellen. Diese Verfahren haben den grossen Nachteil des Einsatzes toxischer oder explosiver Substanzen. Nach den Angaben in Photochemistry and Photobiology *30* (1979), S. 81 bis 87 und Chem. Ber. *115* (1982), S. 3032 bis 3041) kann man Schwefelylide, die bekanntlich von üblichen Oxidationsmitteln nicht angegriffen werden, durch Behandlung mit photochemisch unter Methylenblau-Sensibilisierung erzeugtem Singulett-Sauerstoff oder mit Ozon in die entsprechenden Keto-Verbindungen überführen. Diese Methoden haben den Nachteil eines hohen Energieverbrauchs und des Bedarfs spezieller Apparaturen. Da Schwefelylide leicht zugängliche Ausgangsstoffe darstellen, war nach einem Verfahren zu suchen, das es ermöglicht, diese Ausgangsstoffe auf einfachere Weise in die gesuchten vicinalen Polycarbonylverbindungen zu überführen.

Diese Aufgabe wurde durch das erfindungsgemässe Verfahren gelöst. Nach dem Verfahren der Erfindung kann man vicinale Polycarbonylverbindungen der Formel:

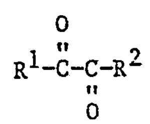

(I)

in der $R^1$ einen Alkyl- oder Arylrest oder einen aliphatischen oder aromatischen Alkohol- oder Aminrest, und $R^2$ einen Alkyl-, Aryl- Carbonamid- oder Esterrest oder einen Rest der Formel $-COX$, in der X für einen Alkyl- oder Arylrest oder einen heterocyclischen Rest steht, bedeuten, dadurch herstellen, dass man Schwefelylide der Formel:

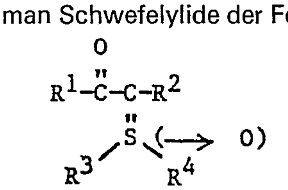

(II)

in der die Reste $R^3$ und $R^4$ jeweils Alkyl- oder Arylreste oder beide Reste $R^3$ und $R^4$ zusammen einen Alkylenrest bedeuten, bei Temperaturen bis 100° C in einem Lösungsmittel mit einer peroxidischen Verbindung behandelt.

Das Verfahren der Erfindung lässt sich formelmässig so beschreiben:

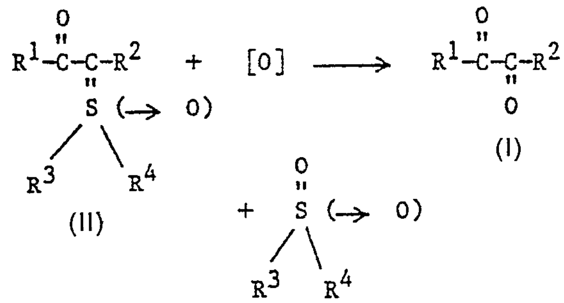

(II) (I) (III)

Die Schwefelylide der Formel (II) sind z. B. nach den in den deutschen Patentschriften Nrn. 1207379 und 1226561 beschriebenen Verfahren erhältlich.

In den Schwefelyliden der Formel (II) bedeuten Alkylreste z. B. Alkylreste mit 1 bis 12, vorzugsweise 1 bis 6 C-Atomen. Arylreste sind z. B. Phenylreste, die z. B. noch durch niedere Alkylreste, wie Methyl- oder Ethylgruppen oder durch Halogenatome substituiert sein können. Aliphatische oder aromatische Alkohol- oder Aminreste sind z. B. Reste der Formeln $-OR^5$ oder $-NR^6R^7$, in denen die Reste $R^5$, $R^6$ und $R^7$ Alkyl oder Arylreste der obengenannten Art bedeuten und $R^6$ auch für ein Wasserstoffatom stehen kann. Carbonamidgruppen sind z. B. Carbonylgruppen oder Reste der Formel $-CONR^6R^7$. Esterreste sind z. B. Reste der Formel $-COOR^5$ und heterocyclische Reste sind z. B. Reste des Furans, Thiophens, Oxazols, Imidazols oder Chinolins.

Als Oxidationsmittel verwendet man peroxidische Verbindungen. Das sind z. B. Wasserstoffperoxid, Peroxysäuren, wie Peressigsäure, m-Chlorperessigsäure, Perameisensäure, Permanganate, wie Kaliumpermanganat und Natriumpermanganat oder Kaliumperchromat.

Man behandelt die Ylide mit den Oxidationsmitteln z. B. bei Temperaturen bis 100° C, vorzugsweise bei −20 bis +40° C. Als Lösungsmittel sind z. B. Wasser, niedermolekulare Alkohole, wie Methanol und Ethanol, niedermolekulare Säuren, wie Essigsäure, Chlorkohlenwasserstoffe, wie Methylenchlorid, Aromaten, wie Benzol, Chlorbenzol oder Toluol, Ether, niedermolekulare Ketone, wie Aceton oder Mischungen dieser Lösungsmittel geeignet. Das Oxidationsmittel gibt man z. B. in stöchiometrischer Menge oder im Überschuss oder Unterschuss zu. Zweckmässigerweise wendet man das Oxidationsmittel in der 1- bis 3-fachen stöchiometrischen Menge an. Das Reaktionsende lässt sich leicht durch dünnschichtchromatographische Analyse des unverbrauchten Ylids bestimmen. Von der Sulfoxid- und Sulfonverbindung der Formel (III) lässt sich die gewünschte Polycarbonylverbindung leicht durch Destillation, Kristallisation oder Extraktion bzw. durch direkte Weiterreaktion zum gewünschten Endprodukt abtennen. Hydrate, Acetale und Perhydroxy-addukte

der Reaktionsprodukts werden gegebenenfalls durch bekannte Methoden gespalten.

Nach dem neuen Verfahren erhält man die vicinalen Polycarbonylverbindungen glatt und in guter Ausbeute. Dieses vorteilhafte Ergebnis war nicht zu erwarten. So ist nämlich im Fortschritte der Chem. Forschung, Band 9 (1968), S. 506 angegeben, dass sich die Schwefelylide der Formel (II) mit Wasserstoffperoxid nicht oxidieren lassen. Ausserdem wird in Photochemistry and Photobiology *30* (1979) auf Seite 82 im letzten Absatz darauf hingewiesen, dass eine Photooxidation der Schwefelylide nicht ohne Sensibilisatoren möglich war.

*Beispiel 1:*

2,5 g (10 mMol) Benzoyl-ethoxy-carbonyl-dimethylsulfonium-methylid werden in 50 ml Methylenchlorid gelöst. Man gibt 1,7 ml (17 mMol) einer 30 gew.-%igen wässerigen Lösung von $H_2O_2$ so zu, dass unter guter Durchmischung und Kühlung mit Eis die Temperatur 25°C nicht übersteigt. Nach weiteren 3 Stunden ist alles Ylid umgesetzt und nur wenig $H_2O_2$ mit KJ/Stärke nachzuweisen. Bei 40°C Badtemperatur wird das Lösungsmittel unter Vakuum abgezogen. Man erhält 3,1 g helles Öl, das neben 18,4 Gew.-% Dimethylsulfoxid 2,2-Gew.-% Dimethylsulfon und 3,6 Gew.-% Benzoesäure, als Hauptprodukt Benzoyloxylsäureethylester enthält.

Ausbeute: 2,33 g (9,23 mMol) (92,3%).

Durch Destillation bei 150 bis 153°C und 13 mbar erhält man das Produkt als orangegelbes Öl.

H-NMR (80 MHz, $CDCl_3$, TMS-Standard)

t 1,37 ppm (3H, $-CH-CH_3$), q 4,45 ppm (2H, $-CH_2-CH_3$), m 7,6 ppm (3H, aromat.), m 8,0 ppm (2H, aromat.).

*Beispiel 2:*

Zu einer Lösung von 5,0 g (20 mMol) Benzoyl-ethoxy-carbonyl-dimethylsulfonium-methylid in 200 ml Methylenchlorid werden bei Kühlung auf 3 bis 5°C innerhalb 80 min 23 mMol Peressigsäure getropft. Man rührt 2 Stunden im Eisbad und anschliessend 10 Stunden bei Zimmertemperatur. Dann engt man bei 35°C am Rotationsverdampfer ein und erhält 10,4 g ein schwach gelb gefärbtes Öl. Durch Destillation bei 150 bis 153°C und 13 mbar erhält man 3,65 g (17,7 mMol) Benzoyloxalsäureethylester (88,5% d. Th.).

Der Ansatz wird mit 126 g (0,5 Mol) des Ylids und 1,26 l Methylenchlorid wiederholt. Nach Abtrennung wird die Methylenchlorid-Phase 2mal mit 150 ml Wasser gewaschen, getrocknet und eingeengt. Man erhält 150,6 g eines gelben Öls, das durch Vakuumdestillation von Essigsäure, Dimethylsulfoxid und Dimethylsulfon befreit wird. Man erhält 96,3 g Benzoyloxalsäureethylester als öliges Produkt (0,426 mol, 85,2% d. Th.).

Setzt man Benzoyloxalsäureethylester entsprechend der in Liebigs Ann. Chem. *758* (1972), S. 173 bis 176 angegebenen Arbeitsvorschrift mit Benzamidrazon um, so erhält man 3,5-Diphenyl-6-ethoxycarbonyl-1,2,4-triazin in 89%iger Ausbeute. Durch anschliessende alkalische Verseifung erhält man daraus Diphenyl-triazincarbonsäure und aus dieser durch thermische Decarboxylierung 3,5-Diphenyl-1,2,4-triazin von Schmp. 98 bis 100°C in 70%iger Ausbeute.

*Beispiel 3:*

24,2 g (0,1 mol) 2-Furoyl-ethoxy-carbonyl-dimethylsulfonium-methylid werden in 200 ml Methylenchlorid gelöst. In diese Lösung tropft man bei 5°C innerhalb von 180 min 0,1 mMol Peressigsäurelösung (Chem. Ber. *114* (1981) S. 1966). Man rührt 12 Stunden bei Raumtemperatur und engt dann bei 40°C am Rotationsverdampfer ein. Der Rückstand von 42 g braunem Öl wird durch fraktionierte Destillation gereinigt. Man erhält 11,6 g (59,2% d. Th.) an 2-Furoyloxalylethylester (Sdp. 145 bis 150°C/0,5 mbar) als gelbes Öl.

H-NMR (80 MHz, $CDCl_3$)

t 1,37 (3H, $-OCH_2CH_3$), q 4,45 ppm 2H ($-OCH_2CH_3$), m 6,60 ppm (1H, Furyl-), m 7,38 ppm (1H, Furyl-), m 7,67 ppm (1H, Furyl-).

Mit 2,4-Dinitrophenylhydrazin im Überschuss entsteht das Bis-hydrazon, rote Kristalle, Schmp. 256 bis 259°C.

*Analyse* für $C_{21}H_{16}N_8O_{11}$ (MG 556,40):

| | | | | |
|---|---|---|---|---|
| Ber.: | C 45,33 | H 2,90 | N 20,23 | O 31,63% |
| Gef.: | C 45,1 | H 3,1 | N 20,1 | O 31,7 % |

*Beispiel 4:*

4,38 g (20 mMol) N,N-Dimethyl-carbamoyl-ethoxycarbonyl-dimethylsulfonium-methylid werden in 50 ml Methylenchlorid gelöst und mit 4 mMol Peressigsäure analog Beispiel 3 oxidiert.

Man erhält durch Einengen und fraktionierte Destillation im Kugelrohr Mesoxalsäuremonoethylester-dimethylamid.

H-NMR (80 MHz, $CDCl_3$)

t 1,30 (3H, $-OCH_2CH_3$), s 3,10 (6H, $NCH_3$), q 4,40 (2H, $-OCH_2CH_3$).

Das auf üblichen Wege dargestellte 2,4-Dinitrophenylhydrazon (Schmp. 217 bis 220°C, $CH_3OH$) ergibt folgende Analysenwerte:

*Analyse* für $C_{13}H_{15}N_5O_7$ (MG 353,29):

| | | | | |
|---|---|---|---|---|
| Ber.: | C 44,20 | H 4,28 | N 19,82 | O 31,70% |
| Gef.: | C 44,3 | H 4,40 | N 20,0 | O 31,4 % |

*Beispiel 5:*

2,85 g (10 mMol) Dibenzoyl-dimethylsulfonium-methylid werden in 30 ml Methylenchlorid gelöst. Unter Kühlung auf 3°C gibt man innerhalb von 30 min tropfenweise 17 mMol Peressigsäure hinzu. Die Lösung färbt sich in deutlich exothermer Reaktion gelb. Man lässt langsam auf Raumtemperatur kommen. Nach 10 Stunden wird die im Reaktionsansatz nachweisbare Restmenge Ylid (DC-Kontrolle) durch Zugabe von weiteren 2,5 mMol Peressigsäure oxidiert (2 Stunden und Zimmertemperatur). Man engt den Ansatz unter Vakuum ein und erhält 4,0 g rötlich gelbes Öl. Durch mehrfache Extraktion mit siedendem Cyclohexan trennt man von Dimethylsulfon ab, engt erneut ein und erhält 1,1 g 1,3-Diphenylpropantrion als orangefarbene Kristalle (45% d. Th.) mit dem Schmp. 68 bis 70°C (Lit. 67 bis 70°C).

*Analyse* für $C_{15}H_{10}O_3$ (MG 238,24):

| | | | |
|---|---|---|---|
| Ber.: | C 75,62 | H 4,23 | O 20,15% |
| Gef.: | C 75,8 | H 4,4 | O 19,8 % |

*Beispiel 6:*

27 g (100 mMol) N-Phenylcarbamoyl-ethoxycarbonyl-dimethylsulfonium-methylid werden in 500 ml Methylenchlorid gelöst. Man kühlt auf 3°C ab (Eisbad) und tropft 17 ml (170 mMol) Perhydrol (eine 30%ige wässerige Lösung von $H_2O_2$) rasch zu. Man rührt den Ansatz 24 Stunden bei Raumtemperatur und engt dann bei 30°C am Rotationsverdampfer unter Vakuum (20 mbar) ein. Man löst den Rückstand in 200 ml Ethanol und saugt vom abgeschiedenen Nebenprodukt Diphenylharnstoff ab. Die Gehaltsbestimmung des gebildeten Mesoxalsäuremonoanilidmonoethylesters erfolgt über das 2,4-Dinitrophenylhydrazon. Man erhält 27 g gelbes Kristallisat (63,7% d. Th.) vom Schmp. 208 bis 210°C (aus Methanol/Methylenchlorid).

H-NMR (80 MHz, $CDCl_3$)

t 1,47 ppm (3H, $-OCH_2-CH_3$), q 4,45 ppm (2H, $-OCH_2-CH_3$), m 7,0 bis 8,6 ppm (8H aromat. Protonen), 11,0 ppm und 15,8 ppm (je 1NH).

*Analyse* für $C_{17}H_{15}N_5O_7$ (MG 401,33):

| | | | | |
|---|---|---|---|---|
| Ber.: | C 50,88 | H 3,77 | N 17,45 | O 27,91% |
| Gef.: | C 50,4 | H 3,6 | N 17,9 | O 28,1 % |

Auf entsprechende Weise, jedoch mit Peressigsäure, m-Chlorperbenzoesäure und Kaliumpermanganat als Oxidationsmittel, werden ähnliche Resultate erhalten.

*Beispiel 7:*

Zu einer Lösung von 5,7 g (30 mMol) Acetylethoxy-carbonyl-dimethylsulfonium-methylid in 30 ml Wasser werden bei 5°C 30 mMol einer 30%igen. wässerigen $H_2O_2$-Lösung tropfenweise zugegeben. Nach 18 Stunden bei Raumtemperatur erhält der Ansatz nach NMR-Analyse zu 30% unverbrauchtes Ylid und zu 70% 2 Keto-acetessigester (sowie 35% Dimethylsulfoxid und 35% Dimethylsulfon). Das Oxidationsprodukt wird als Bis-2,4-dinitrophenylhydrazon charakterisiert (Schmp. 238 bis 242°C, $CH_3OH/H_2O$).

*Analyse* für $C_{18}H_{16}N_8O_{10}$ (MG 504,37):

| | | | | |
|---|---|---|---|---|
| Ber.: | C 42,86 | H 3,20 | N 22,22 | O 31,70% |
| Gef.: | C 43,1 | H 3,9 | N 21,9 | O 31,1 % |

*Beispiel 8:*

Zu einer Lösung von 22 g (100 mMol) Diethoxycarbonyl-dimethylsulfonium-methylid in 500 ml Methylenchlorid gibt man bei 5°C innerhalb von 2 Stunden eine Lösung von 45 g 80%ige m-Chlorperbenzoesäure (200 mMol) in 1000 ml Methylenchlorid. Dabei werden Ylid und Persäure vollständig umgesetzt. Man engt ein und trennt mit Tetrachlorkohlenstoff vom kristallisierten Dimethylsulfon ab. Die Lösung wird eingeengt. Der Extrakt enthält m-Chlorbenzoesäure und Mesoxalsäurediethylester, der zur Ausbeutebestimmung in das 2,4-Dinitrophenylhydrazon umgewandelt wird. Man erhält 35,0 g feine, gelbe Nadeln vom Schmp. 115 bis 117°C (Lit., 117 bis 118°C). Das entspricht einer Ausbeute von 98% d. Th.

*Beispiel 9:*

15,8 g (100 mMol) Kaliumpermanganat werden in 40 ml 4 n Schwefelsäure gelöst. Das Gemisch wird mit 200 ml Methylenchlorid intensiv gerührt und auf 10°C gekühlt. Man gibt nun eine Lösung von 5,7 g 2-Thenoyl-ethoxycarbonyl-tetramethylensulfoniummethylid in Methylenchlorid rasch hinzu, wobei man durch Kühlung eine Reaktionstemperatur unter 30°C einhält. Nach 15 min wird vom ausgefallenen Braunstein abgesaugt und die Wasserphase abgetrennt.

Die Methylenchloridphase ergibt nach Trocknen und Einengen 5,6 g rohen 2-Thenyloxalylethylester als gelbes Öl. Zur Gehaltsbestimmung gibt man 2,2 g (20,2 mMol) o-Phenylendiamin in warmem Ethanol zu. Nach 4 Stunden erhält man 3,7 g (65% bez. auf eingesetztes Ylid) 2-Ethoxycarbonyl-3-(2-thenyl)-chinoxalin als gelbe Nadeln mit Schmp. 153 bis 155°C (EtOH).

*Analyse* für $C_{15}H_{12}N_2O_2S$ (MG 284,33):

| | | | | |
|---|---|---|---|---|
| Ber.: | C 63,26 | H 4,25 | N 9,85 | O 11,25 |
| | S 11,27% | | | |
| Gef.: | C 63,3 | H 4,4 | N 9,9 | O 11,3 |
| | S 11,3 % | | | |

H-NMR (80 MHz, $CDCl_3$)

t 1,37 (3H, $-OCH_2CH_3$); q 4,50 ppm (2H, $-OCH_2CH_3$); m 7,15 ppm (1H, Thiophen); m 7,50 ppm (2H, Thiophen); m 7,75 ppm (2H), und m 8,10 ppm (2H) für den Chinoxalinring.

*Beispiel 10:*

Man verfährt analog Beispiel 9, wobei man jedoch 365 g (10 mMol) N-Phenylcarbamoyl-N'-p-chlorphenyl-carbamoyl-dimethyloxosulfoniummethylid mit 950 g Kaliumpermanganat oxidiert. Man isoliert 170 g N-Phenyl-N'-p-chlorphenyl-mesoxalsäureanilid in Form des Halbacetals (48% d. Th.) nach Umlösen aus Ethanol in Form gelber Kristalle.

*Analyse* für $C_{17}H_{17}ClN_2O_4$ (MG 348,79):

| | | | | |
|---|---|---|---|---|
| Ber.: | C 58,54 | H 4,91 | N 8,03 | O 18,35 |
| | Cl 10,16% | | | |
| Gef.: | C 58,4 | H 5,0 | N 8,1 | O 18,4 |
| | Cl 10,1 % | | | |

*Beispiel 11:*

Analog Beispiel 9 werden unter intensivem Rühren und Kühlung im Eisbad 27,8 g (0,1 Mol) Benzoyl-carbethoxy-tetramethylensulfoniummethylid mit 63,2 g (0,4 Mol) Kaliumpermanganat in 200 ml 4 n Schwefelsäure und 200 ml Methylenchlorid oxidiert. Während der Zugabe des in 100 ml Methylenchlorid gelösten Ylids beobachtet man eine stark exotherme Reaktion und die Abscheidung von Braunstein. Man rührt 15 min

nach, wobei man eine Lösung von Benzoyloxalsäureethylester erhält. Ohne diesen Ester zu isolieren, gibt man 10,8 g (0,1 Mol) o-Phenylendiamin hinzu. Nach 1 Stunden filtriert man und wäscht den Filterkuchen gut mit Methylenchlorid. Die organische Phase wird abgetrennt, getrocknet und eingeengt. Das erhaltene Öl (30,1 g) wird mit 50 ml 2 n NaOH in 50 ml Ethanol verseift. Der Alkohol wird im Vakuum abdestilliert und die wässerige Phase mit Methylenchlorid gewaschen. Durch Zugabe von 2 n Salzsäure wird die 3-Phenyl-chinoxalin-2-carbonsäure gefällt. Man erhält 15,8 g (63% d. Th., bezogen auf eingesetztes Ylid) Reinprodukt (dünnschichtchromatographische Kontrolle) als helles Pulver mit Zersetzungspunkt 160°C.

*Analyse* für $C_{15}H_{10}N_2O_2$ (MG 250,26):

| | | | | |
|---|---|---|---|---|
| Ber.: | C 71,99 | H 4,03 | N 11,19 | O 12,79% |
| Gef.: | C 71,7 | H 4,1 | N 11,5 | O 12,7 % |

*Beispiel 12:*

Man lässt in ein gut gerührtes Gemisch aus 63,2 g (0,4 Mol) Kaliumpermanganat, 200 ml 2 n Schwefelsäure und 500 ml Methylenchlorid bei 20 bis 25°C (Eiskühlung) eine Lösung von 19,4 g (0,1 Mol) Acetyl-phenyl-dimethylsulfonium-methylid in 150 ml Methylenchlorid innerhalb von 15 min einlaufen. Eine deutliche exotherme Reaktion und die spontane Bildung von Braunstein ist zu beobachten. Nach Zugabe rührt man noch weitere 15 min bei der angegebenen Temperatur. Die DC-Reaktionskontrolle (System $SiO_2$, n-Butanol-Wasser-Essigsäure: 4:1:1) zeigt zu diesem Zeitpunkt das Ausgangsmaterial mit dem Rf 0,25 als nicht vorhanden.

Zur Abtrennung des Braunsteins und des überschüssigen Kaliumpermanganats wird die braune Suspension abgesaugt. Der Rückstand wird gut mit Methylenchlorid gewaschen. Die Wasserphase im Filtrat wird abgetrennt und einmal mit Methylenchlorid extrahiert. Die vereinigten organischen Lösungen werden über wasserfreiem Natriumsulfat getrocknet. Nach der Filtration erhält man eine hellbraune Lösung, die das gebildete 1-Phenyl-1,2-propandion enthält. 10,8 g (0,1 mol) o-Phenylendiamin werden zugegeben. Das Reagenz löst sich schnell auf, die Lösung wird dunkelbraun und 2 bis 3 min später trennt sich Wasser ab. Nach der DC (System $SiO_2$, Methylenchlorid) ist nach 10 min das 1-Phenyl-1,2-propandion mit Rf 0,56 vollständig zu 2-Methyl-3-phenylchinoxalin mit Rf 0,21 umgesetzt.

*Beispiel 13:*

0,56 g (2 mMol) Phenacyl-ethoxycarbonyltetramethylen-sulfoniummethylid werden in einem Gemisch aus 20 ml 4 n Schwefelsäure und 100 ml Methylenchlorid verteilt. Die Mischung wird durch Eiskühlung auf 10°C gekühlt. Bei guter Durchmischung werden portionsweise innerhalb 20 min 10,5 g (35 mMol) Kaliumperchromat eingetragen. Zwischen den Zugaben wird gewartet, bis die Sauerstoffentwicklung aus der Zersetzung des Oxidationsmittels abgeklungen ist. Das Gemisch wird türkisfarben. Die Reaktionstemperatur wird durch Eiskühlung bei 15 bis 20°C gehalten. Nach beendeter Zugabe des Perchromats lässt man 15 min nachrühren. Die blaue Methylenchlorid-Phase wird abgetrennt und die saure, wässerige Lösung wird fünfmal mit je 20 ml Methylenchlorid extrahiert. Die vereinigten Extrakte werden über wasserfreiem Natriumsulfat getrocknet. Das Dünnschichtchromatogramm (System $SiO_2$, Methylenchlorid und 5% Isopropanol) zeigt den gebildeten 2,3-Dioxo-3-phenylpropansäureethylester (Rf 0,42) neben einem Rest des Ausgangsmaterials (Rf 0,27) an. Zur filtrierten Lösung werden 0,22 g (2 mMol) o-Phenylendiamin gegeben. Man lässt eine Stunde bei Raumtemperatur stehen. Nach dünnschichtchromatographischer Reaktionskontrolle ist der Diketoester vollständig zum 2-Phenylchinoxalin-3-carbonsäureethylester (Rf 0,70) umgesetzt. Es lassen sich 0,22 g (0,88 mMol) 2-Phenylchinoxalin-3-carbonsäure isolieren (44% d. Th.), Fp. 162°C Zers.

*Analyse* für $C_{15}H_{10}N_2O_2$ (MG 250,26):

| | | | | |
|---|---|---|---|---|
| Ber.: | C 71,99 | H 4,03 | N 11,19 | O 12,79% |
| Gef.: | C 71,7 | H 4,1 | N 11,4 % | |

*Beispiel 14:*

In ein gut gerührtes Gemisch aus 12,7 g (80 mMol) Kaliumpermanganat, 50 ml 4 n Schwefelsäure und 200 ml Methylenchlorid werden bei 15 bis 18°C 8,3 g (40 mMol) Phenylacetyl-dimethylsulfoniumbromid innerhalb 5 min eingetragen. Durch Eiskühlung ist die exotherme Reaktion leicht zu beherrschen. Nach beendeter Zugabe wird das Eisbad entfernt. Man rührt noch 20 min bei 20 bis 22°C. Der entstandene Braunstein und überschüssiges Kaliumpermanganat werden abgesaugt. Der Rückstand wird gut mit Methylenchlorid gewaschen. Um überschüssiges Permanganat im Filtrat zu zerstören, wird unter Rühren etwa 1 g Oxalsäure zugegeben. Nach einigen Minuten ist die $CO_2$-Entwicklung beendet und die Färbung beseitigt. Die wässerige Phase der Reaktionslösung wird abgetrennt und zweimal mit Methylenchlorid extrahiert. Die vereinigten organischen Phasen werden über wasserfreiem Natriumsulfat getrocknet und nach der Filtration am Rotationsverdampfer bei 40°C im Vakuum eingeengt. Man erhält einen Rückstand von 5,5 g eines braunen Öls. Nach der Dünnschichtchromatographie (System $SiO_2$, Methylenchlorid) ist als Hauptkomponente mit Rf 0,56 das 1-Phenyl-1,2-dioxopropan neben mehreren geringen Verunreinigungen angezeigt. Das Rohprodukt wird in 20 ml Methylenchlorid gelöst und mit 3,2 g (30 mMol) o-Phenylendiamin versetzt. Die homogene, dunkelbraune Lösung lässt man über Nacht bei Raumtemperatur stehen. Die dünnschichtchromatographische Kontrolle zeigt den vollständigen Umsatz des Diketons zum 2-Methyl-3-phenylchinoxalin (Rf 0,21) an. Man isoliert 2,7 g (12,2 mMol) 2-Methyl-3-phenylchinoxalin (40,7% d. Th.) als kristallisierendes, orangefarbenes Öl, das identisch ist mit dem Produkt aus Beispiel 12.

*Beispiel 15:*

11,0 g (50 mMol) Diethoxycarbonyl-dimethylsulfoniummethylid werden in 25 ml Ethanol und 75 ml Wasser gelöst. Das homogene Gemisch wird im Eisbad auf 5°C gekühlt. Zu der gerührten Lösung werden innerhalb 5 min 17,0 g (150 mMol) Perhydrol (eine 30%ige wässerige Lösung von $H_2O_2$) getropft. Man rührt 16 Stunden nach und lässt dabei langsam bis auf 20°C erwärmen. Die Reaktionskontrolle mittels Dünnschichtchromatographie (System $SiO_2$, Methylenchlorid und 5% Isopropanol) zeigt kein Ausgangsmaterial (Rf 0,33) mehr an. Durch Besprühen mit 2,4-Dinitrophenylhydrazinlösung wird als Hauptprodukt Mesoxalsäurediethylester (Rf 0,45) als gelborangefarbener Fleck entwickelt. Kaliumiodid-Stärke-Papier zeigt noch überschüssiges Peroxid an.

Zur Abtrennung von Ethanol und dem entstandenen Acetaldehyd wird die Reaktionslösung bei 40°C am Rotationsverdampfer im Vakuum auf 55 g eingeengt. Die farblose Lösung wird in einem Eisbad auf 10°C gekühlt und unter Rühren mit Eisen (II)-sulfat versetzt, bis Kaliumiodid-Stärke-Papier kein Oxidationsmittel mehr anzeigt. Die braune Lösung wird 6mal mit je 50 ml Methylenchlorid extrahiert. Die vereinigten Extrakte werden über wasserfreiem Natriumsulfat getrocknet und nach der Filtration am Rotationsverdampfer bei 40°C Badtemperatur im Vakuum eingeengt. Aus dem Rückstand (15,2 g hellbraunes Öl) kristallisiert Dimethylsulfon in Form langer Nadeln. Mit eiskaltem Diethylether wird das Öl vom Kristallisat getrennt. Das Lösungsmittel wird am Rotationsverdampfer abgezogen und das erhaltene Rohprodukt über eine Destillationsbrücke im Wasserstrahlvakuum fraktioniert. Man erhält 6,6 g (38 mMol) = 76% d. Th. Mesoxalsäurediethylester.

*Beispiel 16:*

Eine Lösung von 25,2 g (100 mMol) Benzoylethoxycarbonyl-dimethylsulfoniummethylid in 250 ml Eisessig wird auf 10°C im Eisbad gekühlt. Zur farblosen, homogenen Lösung werden 154 ml Peressigsäurelösung (Persäurekonzentration, 1,3 mMol·ml$^{-1}$) so zugetropft, dass die Innentemperatur 15°C nicht übersteigt. Man rührt 2 Stunden nach. Durch die dünnschichtchromatographische Reaktionskontrolle wird der vollständige Umsatz des Ausgangsmaterials (DC-System: Kieselgel, Methylenchlorid und 5% Isopropanol) festgestellt. Zur Gehaltsbestimmung wird ein aliquoter Teil der Reaktionslösung mit 2,4-Dinitrophenylhydrazinlösung im Überschuss versetzt. Das rote Kristallisat wird als bis-2,4-Dinitrophenylhydrazon des Benzoyloxalsäureethylesters analysiert.

Fp. 217 bis 219°C.

*Analyse* für $C_{23}H_{18}N_8O_{10}$ (MG 566,44):

| | | | | |
|---|---|---|---|---|
| Ber.: | C 48,77 | H 3,20 | N 19,78 | O 28,24% |
| Gef.: | C 48,8 | H 3,3 | N 19,8 | O 28,0 % |

Die Ausbeute beträgt 67% d. Th.

*Beispiel 17:*

Eine Lösung von 0,28 g (1 mMol) Benzoylethoxycarbonyl-tetramethylensulfoniummethylid in 10 ml Benzol wird bei Raumtemperatur mit 0,25 ml Perhydrol 17 Stunden gerührt. Die dünnschichtchromatographische Reaktionskontrolle (System $SiO_2$, Methylenchlorid + 5% Isopropanol) zeigt den vollständigen Umsatz des Ausgangsylids zu Benzoyloxalsäureethylester und wenig Benzoesäure an. (Benzoesäure entsteht unter diesen Reaktionsbedingungen nicht aus Benzol.) Das farblose Gemisch wird über Natriumsulfat getrocknet und am Rotationsverdampfer bei 40°C im Vakuum eingeengt. Der Eindampfrückstand (0,45 g gelbes Öl) wird im Überschuss 2,4-Dinitrophenylhydrazinlösung aufgenommen, woraufhin das gelborange bis-2,4-Dinitrophenylhydrazon des Benzoyloxalsäureethylesters auskristallisiert. Anderntags wird abgesaugt und mit Wasser gewaschen. Nach der Trocknung bei 50°C im Vakuum werden 0,46 g (0,81 mMol = 81% d. Th.) des Benzoyloxalsäureethylester-2,3-bis(2,4-dinitrophenyl)-hydrazons ausgewogen.

Fp. 215 bis 217°.

*Analyse* für $C_{23}H_{18}N_8O_8$ (MG 566,14):

| | | | | |
|---|---|---|---|---|
| Ber.: | C 48,77 | H 3,20 | N 19,78 | O 28,24% |
| Gef.: | C 48,7 | H 3,3 | N 19,7 | O 28,5 % |

*Beispiel 18:*

In 40 ml 1,2-Dimethoxyethan werden 2,8 g (10 mMol) Benzoylethoxycarbonyl-tetramethylensulfoniummethylid gelöst. Die farblose, homogene Mischung wird im Eisbad auf 15°C gekühlt. Unter Rühren wird die Lösung von 5,0 g (~ 23 mMol) einer 80%igen 3-Chlorperbenzoesäure in 20 ml 1,2-Dimethoxyethan innerhalb 20 min so zugetropft, dass die Reaktionstemperatur 20°C nicht übersteigt. Eine leichte Eiskühlung ist dabei erforderlich. Es entsteht eine für den gebildeten Benzoyloxalsäureethylester typische gelbe Lösung. Nach 2 Stunden Rühren bei 20 bis 22°C zeigt die dünnschichtchromatographische Reaktionskontrolle (System $SiO_2$, Methylenchlorid + 5% Isopropanol) den fast vollständigen Umsatz des Ausgangsmaterials an. Zu dem peroxidfreien Gemisch werden 1,1 g (10 mMol) o-Phenylendiamin gegeben. Die nun hellbraune Lösung lässt man 2 Stunden bei Raumtemperatur rühren und engt sie dann am Rotationsverdampfer im Vakuum bei 40°C ein. Der feste Rückstand wird in ca. 100 ml Methylenchlorid gelöst und zweimal mit je 10 ml 2 n Natronlauge und einmal mit Wasser gewaschen. Die Methylenchloridphase wird über Natriumsulfat getrocknet und nach der Filtration des Trockenmittels eingeengt. Man erhält 4,1 g eines hellbraunen Öls, das als Hauptbestandteil den 2-Phenylchinoxalin-3-carbonsäureethylester enthält. Dieser Ester wird mit 20 ml 1 n Natronlauge und 5 ml Ethanol 15 Stunden bei Raumtemperatur gerührt. Am Rotationsverdampfer wird das Gemisch vom Ethanol befreit. Die wässerige Lösung wird mit 2 n Salzsäure neutralisiert. Die 2-Phenylchinoxalin-3-carbonsäure fällt aus. Das hellbraune Produkt wird abgesaugt und mit Wasser gewaschen. Nach der Trocknung im Vakuum bei 70°C verbleiben 1,9 g (7,6 mMol) 2-Phenylchinoxalin-3-carbonsäure (76% d. Th.).

## Patentansprüche

1. Verfahren zur Herstellung von vicinalen Polycarbonylverbindungen der Formel:

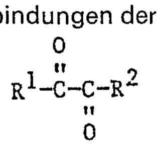 (I)

in der $R^1$ einen Alkyl- oder Arylrest oder einen aliphatischen oder aromatischen Alkohol- oder Aminrest, und $R^2$ einen Alkyl-, Aryl-, Carbonamid- oder Esterrest oder einen Rest der Formel $-COX$, in der X für einen Alkyl- oder Arylrest oder einen heterocyclischen Rest steht, bedeuten, dadurch gekennzeichnet, dass man Schwefelylide der Formel:

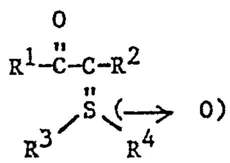 (II)

in der die Reste $R^3$ und $R^4$ jeweils Alkyl- oder Arylreste oder beide Reste $R^3$ und $R^4$ zusammen einen Alkylenrest bedeuten, bei Temperaturen bis 100°C in einem Lösungsmittel mit einer peroxidischen Verbindung behandelt.

2. Verfahren nach Anspruch 1, dadurch gekennzeichnet, dass man als peroxidische Verbindung Wasserstoffperoxid, eine Peroxysäure, ein Permanganat oder Kaliumperchromat verwendet.

## Claims

1. A process for the preparation of a vicinal polycarbonyl compound of the formula:

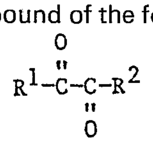 (I)

where $R^1$ is alkyl, aryl or an aliphatic or aromatic alcohol or amine radical, and $R^2$ is alkyl, aryl, carboxamido, an ester radical or a radical of the formula $-COX$, where X is alkyl, aryl or a heterocyclic radical, wherein a sulfur ylide of the formula:

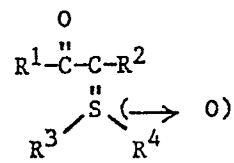 (II)

where $R^3$ and $R^4$ are each alkyl or aryl, or $R^3$ and $R^4$ together form an alkylene radical, is treated, at temperatures up to 100°C in a solvent, with a peroxy compound.

2. A process as claimed in Claim 1, wherein the peroxy compound used is hydrogen peroxide, a peracid, a permanganate or potassium perchromate.

## Revendications

1. Procédé de préparation de composés polycarbonylés vivinaux de la formule:

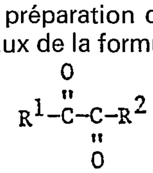 (I)

dans laquelle $R^1$ désigne un groupe alkyle ou aryle ou un reste alcool ou amine aliphatique ou aromatique, et $R^2$ un groupe alkyle, aryle, carbonamide ou ester ou un groupe de la formule $-COX$, où X représente un groupe alkyle ou aryle ou un groupe hétérocyclique, caractérisé en ce que l'on traite des ylures de soufre de la formule:

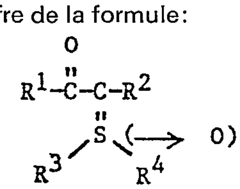 (II)

dans laquelle $R^3$ et $R^4$ désignent chacun un groupe alkyle ou aryle ou forment ensemble un groupe alkylène, dans un solvant et à des températures jusqu'à 100°C, avec un composé peroxydé.

2. Procédé suivant la revendication 1, caractérisé en ce que l'on emploie comme composé peroxydé le peroxyde d'hydrogène, un peroxy-acide, un permanganate ou le perchromate de potassium.